# EUROPEAN PATENT APPLICATION

(11) **EP 4 485 146 A1**
(43) Date of publication of application: **01.01.2025**
(21) Application number: 24182635.3
(22) Date of filing: 17.06.2024
(51) Int. Cl.: G06F 3/01, A61B 3/113, A61B 5/18, B64D 45/00, G06N 3/02, G06N 20/00, G06V 20/59

(54) **PHYSIOLOGY BASED BIO-KINEMATICS MODELING FOR SEGMENTATION MODEL UNSUPERVISED FEEDBACK**

(30) Priority: 30.06.2023 US 202318216911
(71) Applicant: Rockwell Collins, Inc., Cedar Rapids, IA 52498 (US)
(72) Inventor: RADLBECK, Andrew, East Hartford, CT (US); WU, Peggy, East Hartford, CT (US); BLAKESLEE, Brigid A., East Hartford, CT (US); SUNDARAMOORTHI, Ganesh, East Hartford, CT (US)
(74) Representative: Dehns

(57) **Abstract**

A computer system records eye tracking data and identifies movements in the eye tracking data to generate a model of eye movement and pupil dynamics. The model is used to produce a performance metric for the user based on deviations of the predicted output from what is physically possible as defined by the bio-kinematic model. The system continuously stores eye tracking data to enhance the bio-kinematic models. Bio-kinematic models may be generalized or specific to a particular user. The system continuously adjusts and / or weights the bio-kinematic model in real-time based on eye tracking data.

## Description

### BACKGROUND

Data is the driving component for success in machine learning applications. The scarcity of data creates many challenges in the training pilot monitoring systems, and the labor-intensive nature of data collection is a non-trivial problem to solve. The amount of annotated data required to reach suitable performance must be manually generated. A model that must be run on video can be bootstrapped with annotated still images, but the problem remains that, for its intended application, there is no real time way to assess performance.

Consequently, it would be advantageous if an improved apparatus existed that is suitable for gathering data and creating bio-kinematic models for pilot monitoring.

### SUMMARY

In one aspect, embodiments of the inventive concepts disclosed herein are directed to a computer system that records eye tracking data. The system identifies movements in the eye tracking data to generate a model of eye movement and pupil dynamics. The model is used to produce a performance metric for the user based on deviations of the predicted output from what is physically possible as defined by the bio-kinematic model.

In a further aspect, the system continuously stores eye tracking data to enhance the bio-kinematic models. Bio-kinematic models may be generalized or specific to a particular user.

In a further aspect, the system continuously adjusts and / or weights the bio-kinematic model in real-time based on eye tracking data.

It is to be understood that both the foregoing general description and the following detailed description are exemplary and explanatory only and should not restrict the scope of the claims. The accompanying drawings, which are incorporated in and constitute a part of the specification, illustrate exemplary embodiments of the inventive concepts disclosed herein and together with the general description, serve to explain the principles.

### BRIEF DESCRIPTION OF THE DRAWINGS

The numerous advantages of the embodiments of the inventive concepts disclosed herein may be better understood by those skilled in the art by reference to the accompanying figures in which:
FIG. 1 shows a block diagram of a system suitable for implementing embodiments of the incentive concepts disclosed herein;
FIG. 2 shows a flowchart of an exemplary embodiment of the inventive concepts disclosed herein; and
FIG. 3 shows a block diagram of a neural network according an exemplary embodiment of the inventive concepts disclosed herein.

### DETAILED DESCRIPTION

Before explaining at least one embodiment of the inventive concepts disclosed herein in detail, it is to be understood that the inventive concepts are not limited in their application to the details of construction and the arrangement of the components or steps or methodologies set forth in the following description or illustrated in the drawings. In the following detailed description of embodiments of the instant inventive concepts, numerous specific details are set forth in order to provide a more thorough understanding of the inventive concepts. However, it will be apparent to one of ordinary skill in the art having the benefit of the instant disclosure that the inventive concepts disclosed herein may be practiced without these specific details. In other instances, well-known features may not be described in detail to avoid unnecessarily complicating the instant disclosure. The inventive concepts disclosed herein are capable of other embodiments or of being practiced or carried out in various ways. Also, it is to be understood that the phraseology and terminology employed herein is for the purpose of description and should not be regarded as limiting.

**As** used herein a letter following a reference numeral is intended to reference an embodiment of the feature or element that may be similar, but not necessarily identical, to a previously described element or feature bearing the same reference numeral (e.g., 1, 1a, 1b). Such shorthand notations are used for purposes of convenience only, and should not be construed to limit the inventive concepts disclosed herein in any way unless expressly stated to the contrary.

Further, unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of the "a" or "an" are employed to describe elements and components of embodiments of the instant inventive concepts. This is done merely for convenience and to give a general sense of the inventive concepts, and "a' and "an" are intended to include one or at least one and the singular also includes the plural unless it is obvious that it is meant otherwise.

Finally, as used herein any reference to "one embodiment," or "some embodiments" means that a particular element, feature, structure, or characteristic described in connection with the embodiment is included in at least one embodiment of the inventive concepts disclosed herein. The appearances of the phrase "in some embodiments" in various places in the specification are not necessarily all referring to the same embodiment, and embodiments of the inventive concepts disclosed may include one or more of the features expressly described or inherently present herein, or any combination of sub-combination of two or more such features, along with any other features which may not necessarily be expressly described or inherently present in the instant disclosure.

Broadly, embodiments of the inventive concepts disclosed herein are directed to a computer system that records eye tracking data and identifies movements in the eye tracking data to generate a model of eye movement and pupil dynamics. The model is used to produce a performance metric for the user based on deviations of the predicted output from what is physically possible as defined by the bio-kinematic model. The system continuously stores eye tracking data to enhance the bio-kinematic models. Bio-kinematic models may be generalized or specific to a particular user. The system continuously adjusts and / or weights the bio-kinematic model in real-time based on eye tracking data.

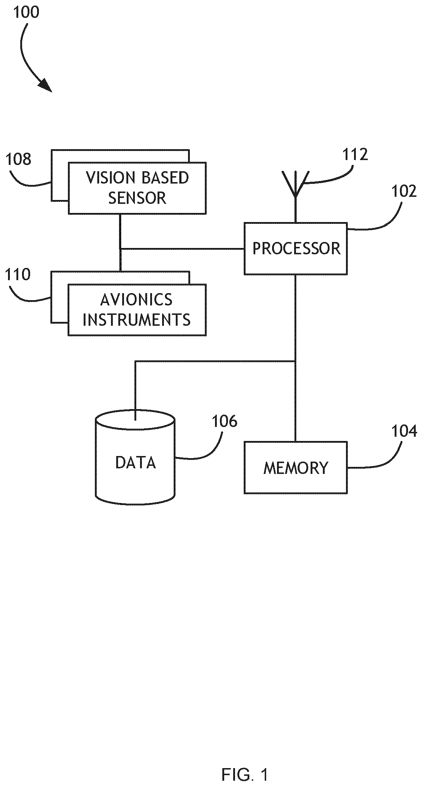

Referring to FIG. 1, a block diagram of a system 100 suitable for implementing embodiments of the incentive concepts disclosed herein is shown. The system 100 includes a processor 102, memory 104 in data communication with the processor 102 for storing processor executable code, and one or more eye tracking sensors / cameras 108 for receiving eye tracking data stream. In at least one embodiment, the eye tracking sensors 108 record eye movement / gaze of a pilot and eye lid position. The processor executable code configures the processor 102 to continuously log the eye tracking data in a data storage element 106. The processor 102 analyzes the eye tracking data to generate a bio-kinematic model of eye movement and pupil dynamics (i.e., a model of physical movement defined by the properties and abilities of bone, muscles, tendons, etc.). The bio-kinematic model may define physical limitations of eye movement, either generally or for a specific user.

In at least one embodiment, the processor 102 may correlate eye tracking data with discreet portions of a training scenario / task that may be defined by a set of steps stored in the data storage element 106. The processor 102 may define metrics for each step that can be characterized via the eye tracking data with respect to the bio-kinematic model; for example, each step may define a scan pattern of instruments that the user should observe. Eye movement may be difficult to detect or characterize generally, and in absolute terms. By comparing eye movements to a bio-kinematic model defined for the specific task, and with respect to specific limitations of the user as defined by the model, faster and more accurate eye movement determinations can be generated.

In at least one embodiment, the discreet portions of the training scenario / task may be associated with known sets of eye movements and pupil dynamics as defined by an expert in the field (e.g., an expert pilot). By recording the user's eye movement and pupil dynamics and correlating that data with corresponding previously sets of expert data, the processor 102 may automatically annotate the eye movement and pupil dynamics data for including in a training data set without the intervention of a outside observer.

In at least one embodiment, the processor 102 may receive specific stimuli such as instrument readings, alerts, or the like, and include such data in the training data set. Embodiments of the present disclosure are directed toward mechanisms for compiling training data to construct a model of movement coincident with specific scenarios to characterize a user's later performance in similar scenarios.

In at least one embodiment, the processor 102 may compare the eye tracking data to an existing bio-kinematic model to characterize the user's performance while simultaneously incorporating the eye tracking data into the training data set.

In at least one embodiment, the processor 102 transfers the stored eye tracking data and other correlated system and task data to an offline storage device for later analysis and correlation to historic data and other outside factors such as crew rest, crew sleet rhythms, flight schedules, etc. Such transfer may be in real time via the wireless communication device 112.

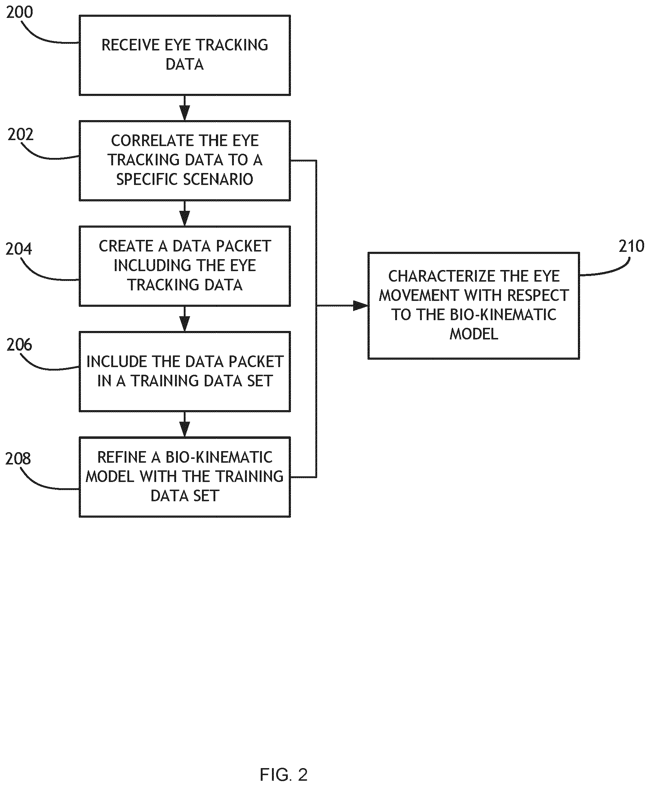

Referring to FIG. 2, a flowchart of an exemplary embodiment of the inventive concepts disclosed herein is shown. A computer system implementing embodiments of the inventive concepts disclosed herein receives 200 an image stream corresponding to eye tracking data from one or more vision-based sensors. The eye tracking data is correlated 202 to a specific flight task or an individual duty schedule of the user / pilot, packaged 204 into a data packet. The data packet is included 206 into a training data set to create or refine 208 a bio-kinematic model of eye movement and pupil dynamics.

In at least one embodiment, the system may contemporaneously characterize 210 the eye tracking data to produce a gaze estimate and scan pattern estimate. The gaze and scan pattern estimates are determined with respect to a bio-kinematic model that may be continuously refined 208 while in use.

Scan patterns may be more accurately characterized with reference to limitations defined by the bio-kinematic model. The bio-kinematic model may be specific to the user.

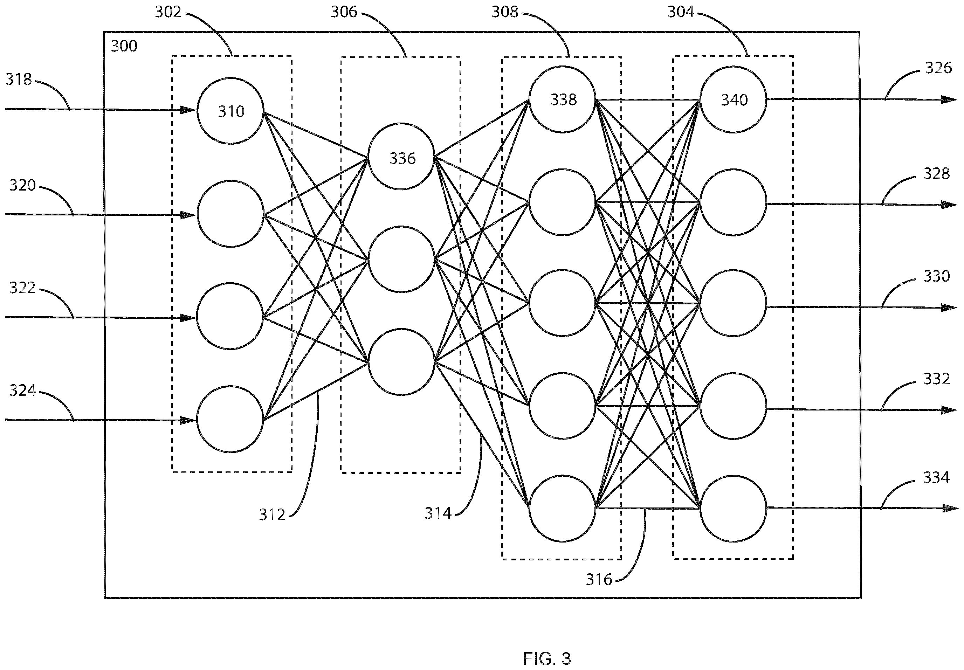

Referring to FIG. 3, a block diagram of a neural network 300 embodying a bio-kinematic model according an exemplary embodiment of the inventive concepts disclosed herein is shown. The neural network 300 comprises an input layer 302 that receives external inputs (including eye tracking data, avionics data, and potentially user or task specific profiles), an output layer 304, and a plurality of internal layers 306, 308. Each layer comprises a plurality of neurons or nodes 310, 336, 338, 340. In the input layer 302, each node 310 receives one or more inputs 318, 320, 322, 324 corresponding to a digital signal and produces an output 312 based on an activation function unique to each node 310 in the input layer 302. An activation function may be a Hyperbolic tangent function, a linear output function, and / or a logistic function, or some combination thereof, and different nodes 310, 336, 338, 340 may utilize different types of activation functions. In at least one embodiment, such activation function comprises the sum of each input multiplied by a synaptic weight. The output 312 may comprise a real value with a defined range or a Boolean value if the activation function surpasses a defined threshold. Such ranges and thresholds may be defined during a training process. Furthermore, the synaptic weights are determined during the training process.

Within the context of the present application, synaptic weights are determined, at least in part, from training data derived from actual eye tracking data recorded during a training scenario or task. The eye tracking data is correlated to the task, and potentially to deviations from predefined expected movements. During training, synaptic weights are iteratively computed and tested against separate data sets.

It is believed that the inventive concepts disclosed herein and many of their attendant advantages will be understood by the foregoing description of embodiments of the inventive concepts disclosed, and it will be apparent that various changes may be made in the form, construction, and arrangement of the components thereof without departing from the broad scope of the inventive concepts disclosed herein or without sacrificing all of their material advantages; and individual features from various embodiments may be combined to arrive at other embodiments. The form herein before described being merely an explanatory embodiment thereof, it is the intention of the following claims to encompass and include such changes. Furthermore, any of the features disclosed in relation to any of the individual embodiments may be incorporated into any other embodiment.

## Claims

1. A computer apparatus comprising:
at least one eye tracking camera (108); and
at least one processor (102) in data communication with a memory storing processor executable code; and
wherein the processor executable code configures the at least one processor (102) to:
receive an image stream from the at least one eye tracking camera (108);
include the eye tracking data into a training set or eye tracking data; and
produce a bio-kinematic model of eye movement via a machine learning algorithm trained on the training set.

2. The computer apparatus of Claim 1, wherein the processor executable code further configures the at least one processor (102) to characterize a user's eye movement to identify gaze and scan pattern with respect to the bio-kinematic model.

3. The computer apparatus of Claim 1 or 2, wherein:
the processor executable code further configures the at least one processor (102) to receive a training scenario or task; and
producing the bio-kinematic model is further based on the training scenario or task.

4. The computer apparatus of Claim 3, wherein the training scenario or task includes predicted eye movement, or wherein the processor executable code further configures the at least one processor (102) to annotate the training set according to the training scenario or task.

5. The computer apparatus of any preceding Claim, wherein the bio-kinematic model defines user specific physical limitations, or wherein the processor executable code further configures the at least one processor (102) as a machine learning neural network embodying the bio-kinematic model.

6. A method comprising:
receiving an image stream from at least one eye tracking camera;
including the eye tracking data into a training set or eye tracking data; and
producing a bio-kinematic model of eye movement via a machine learning algorithm trained on the training set.

7. The method of Claim 6, further comprising characterizing a user's eye movement to identify gaze and scan pattern with respect to the bio-kinematic model.

8. The method of Claim 6 or 7, further comprising receiving a training scenario or task, wherein producing the bio-kinematic model is further based on the training scenario or task.

9. The method of Claim 8, wherein the training scenario or task includes predicted eye movement, or further comprising annotating the training set according to the training scenario or task.

10. The method of Claim 6, wherein the bio-kinematic model defines user specific physical limitations.

11. A pilot monitoring system comprising:
at least one eye tracking camera; and
at least one processor (102) in data communication with a memory storing processor executable code; and
wherein the processor executable code configures the at least one processor (102) to:
receive an image stream from the at least one eye tracking camera;
include the eye tracking data into a training set or eye tracking data; and
produce a bio-kinematic model of eye movement via a machine learning algorithm trained on the training set.

12. The pilot monitoring system of Claim 11, wherein the processor executable code further configures the at least one processor (102) to characterize a user's eye movement to identify gaze and scan pattern with respect to the bio-kinematic model.

13. The pilot monitoring system of Claim 11, wherein:
the processor executable code further configures the at least one processor (102) to receive a training scenario or task; and
producing the bio-kinematic model is further based on the training scenario or task.

14. The pilot monitoring system of Claim 13, wherein the training scenario or task includes predicted eye movement, or wherein the processor executable code further configures the at least one processor (102) to annotate the training set according to the training scenario or task.

15. The pilot monitoring system of Claim 11, wherein the bio-kinematic model defines user specific physical limitations, or wherein the processor executable code further configures the at least one processor (102) as a machine learning neural network embodying the bio-kinematic model.
